# EUROPEAN PATENT SPECIFICATION

(11) **EP 3 243 509 B1**
(45) Date of publication and mention of the grant of the patent: **17.04.2019**
(21) Application number: 17170093.3
(22) Date of filing: 09.05.2017
(51) Int. Cl.: A61K 31/122, A61K 31/704, A61K 31/7084, A61P 25/28

(54) **OROBUCCAL ABSORPTION COMPOSITION FOR NEURO-PROTECTION**
OROBUKKALE ABSORPTIONSZUSAMMENSETZUNG FÜR NEUROSCHUTZ
COMPOSITION À ABSORPTION ORO-BUCCALE POUR LA NEUROPROTECTION

(30) Priority: 09.05.2016 IT UA20163269
(43) Date of publication of application: 15.11.2017
(73) Proprietor: Graal S.r.l., 20900 Monza (IT)
(72) Inventor: SENECI, Antonio, 20900 Monza (MB) (IT); PANFOLI, Isabella, 20900 Monza (MB) (IT); SANTI, Cesare, 20900 Monza (MB) (IT)
(74) Representative: Tagliafico, Giulia

(56) References cited:
- WO-A1-2006/018294
- US-A1- 2011 288 132
- Anonymous: "PROGALIN Italia -Galattosio Purissimo per il Benessere del Cervello", Progalinitalia.com, 30 March 2016 (2016-03-30), XP055408283, Retrieved from the Internet: URL:https://web.archive.org/web/2016033023 0244/http://www.progalinitalia.com/#expand [retrieved on 2017-09-20]

## Description

### FIELD OF THE INVENTION

The present invention relates to an orobuccal absorption composition for the neuro-protection containing D-galactose in association with coenzyme Q10.

### BACKGROUD OF THE INVENTION

Myelin, the lipid coating of some nerves, enables fast transmission of the impulses. It has been shown that myelin has an energy function, being able to use glucose (the main nutrient of the brain) by converting chemical energy into ATP. The latter is sent to the axon to support nerve conduction through gap junctions. This metabolic role is added to the traditional role of "electric insulator" attributed to myelin, today in the process of reconsideration (R. D. Fields, White matter matters, Sci. Am. 298 (2008) 42-49). This discovery has led a group of researchers in the Department of Pharmacy, University of Genoa (DIFAR) to undertake a research on the possible aetiology of multiple sclerosis (MS), of Charcot-Marie Tooth 1A syndrome (CMT1A) and of Amyotrophic Lateral Sclerosis (ALS, motor neuron disease) following the recent hypothesis of a hypometabolism in these neurodegenerative diseases: myelin of patients with multiple sclerosis as well as that in the rat model of CMT1A and in the ALS mouse model show reduced bioenergy capacity. Such researches are documented by more than 30 publications in international scientific journals (see: www.biochemlab.it).

D-galactose (D-gal) is a sugar, which is part of our diet. Determination of metabolites of galactose conducted in various rat organs have established that, in addition to the liver, especially the brain metabolizes D-gal (M. Roser, D. Josic, M. Kontou, K. Mosetter, P. Maurer, W. Reutter, Metabolism of galactose in the brain and liver of rats and its conversion into glutamate and other amino acids., J. Neural Transm. 116 (2009) 131-92)

Coherently, some studies have shown that the D-gal is a good nutrient of isolated myelin, which metabolizes it more efficiently than glucose. Considering that in humans as in many mammals, myelination begins after birth (H.C. Kinney, B.A. Brody, A.S. Kloman, F.H. Gilles, Sequence of central nervous system myelination in human infancy. II. Patterns of myelination in autopsied infants, J. Neuropathol. Exp. Neurol. 47 (1988) 217-34), it is reasonable to think that the D-gal is present in the breast milk to promote first the development of the myelin sheath, contributing to the formation glycolipids (J. Marcus, B. Popko, Galactolipids are molecular determinants of myelin development and axo-glial organization, Biochim. Biophys. Acta. 1573 (2002) 406-13) and later the functionality.

The hypothesis, which is emerging, is that, in addition to being a component of galactocerebrosides, D-gal can also exert a metabolic role, as a substrate for ectopic oxidative phosphorylation described for myelin. In fact, D-gal crosses the blood-brain barrier and enters the nerve cell through GLUT3, a non-insulin-dependent transporter (A.L. Olson, J.E. Pessin, Structure, function, and regulation of the mammalian facilitative glucose transporter gene family, Annu.Rev. Nutr. 16 (1996) 235-56).

Two very recent publications deal with the galactose-myelin correlation (S. Ravera, M. Bartolucci, D. Calzia, A. Morelli, I. Panfoli, Galactose and Hexose 6- Phosphate Dehydrogenase Support the Myelin Metabolic Role, PARIPEX-Indian J. Res. IV (2015) 397-400 and S. Ravera, I. Panfoli, Role of myelin sheath energy metabolism in neurodegenerative diseases, Neural Regen. Res. 10 (2015) 1570).

It is interesting to note how the amount of D-gal ingested with milk is relevant: lactose (disaccharide of milk formed in equal parts of glucose and galactose) is contained in cow's milk to an extent of about 46 gr/litre, therefore with 100 ml of milk, about 2.3 grams of galactose are ingested. However, the latter is not in a free form and, in order to be make it available, it is necessary that the lactose is hydrolysed by the lactase enzyme to glucose and galactose. It is known that a fair percentage of adults has lost a good functionality of the lactase enzyme, moreover often those suffering from neuropathies or autoimmune diseases have a dietary restriction on milk and dairy products, resulting in a lack of galactose, which may not be irrelevant, especially in diseases in which there is demyelination and subsequent reformation of myelin.

It is also known from the scientific literature that galactose has a beneficial effect on some neurodegenerative diseases. Already in 1957, Hartstein and Ullet had observed that the administration of D-gal in part intravenously in part per os in patients with multiple sclerosis (MS) improved the symptoms of the disease (J. Hartstein, G.A. Ulett, Galactose treatment of multiple sclerosis; a preliminary report., Dis. Nerv. Syst. 18 (1957) 255-8).

A beneficial effect of the oral treatment with D-gal has been demonstrated in a rat model with Alzheimer disease (M. Salkovic-Petrisic, J. Osmanovic-Barilar, A. Knezovic, S. Hoyer, K. Mosetter, W. Reutter, Long-term oral galactose treatment prevents cognitive deficits in male Wistar rats treated intracerebroventricularly with streptozotocin, Neuropharmacology. 77 (2014) 68-80). In said model the oral intake of D-gal can have beneficial effects on the learning capacity and memory.

International patent application WO2006/018294 pharmaceutical compositions, which comprise galactose and/or at least one galactose derivative and at least one additive, selected from at least a selenium compound and vitamin E, and optionally one or more magnesium salts. These compositions are reported to be useful in the prophylaxis and therapy of metabolic stress conditions associated with cellular glucose deficiency, especially in stress conditions of the nervous system or in diabetes mellitus.

US 2011/0288132 mentions a formulation comprising cocoa powder, a lipid (e.g. cocoa butter) and nicotine, wherein such formulation is adapted for rapid buccal absorption of nicotine, while not causing local irritation. Galactose is mentioned among the further possible excipients.

A product based on pure D-gal is commercially available (Progalin™), of which an oral administration of 3 g three times per day orally (9 grams per day) alone or in combination with 50 mg of coenzyme Q10 three times per day (150 mg per day) is recommended (see http://www.progalinitalia.com/). Progalin™ is commercially available in the form of powder in jars of 250 or 500 grams.

It is known, however, that D-galctose administered orally is converted into glucose by the liver through the action of two enzymes.

### DESCRIPTION OF THE INVENTION

The applicant who has now found that, if the D-galactose is administered by orobuccal (or peribuccal or buccal) or sublingual way, its efficacy at central nervous system level increases surprisingly, so as to show clear clinical results on patients suffering from chronic-degenerative diseases associated with altered mitochondrial function.

Therefore, the main object of the present invention is a composition formulated for buccal, peribuccal or orobuccal administration, or, in the alternative, for sublingual administration, comprising D-galactose in association with coenzyme Q10 and, optionally, reduced glutathione, as active ingredients, together with pharmaceutically acceptable excipients, for use as food supplement or functional food or food for special medical purposes or as medicament.

The recommended dose for the tablets of the invention corresponds to D-galactose from 250 mg to 1,5 gram, preferably 1 gram, divided in two or three times per day in association with coenzyme Q10 from 25 mg to 150 mg per day, preferably 100 mg, and, optionally with reduced glutathione from 25 mg to 250 mg per day, preferably 100mg.

Therefore the preferred composition according to the invention comprises 0.5 g of D-galactose in association with 50 mg of coenzyme Q10 and, optionally, 50 mg of reduced glutathione, as single dose. This composition may be administered from one to three times per day, preferably two times per day.

The intake of these nutrients has no side effects: in fact, D-gal is normally already present in the human diet. Furthermore, its use is also tested in patients with type II diabetes, and there is in this regard a consolidated experience about the non-toxicity of this product at the indicated doses.

Another object of the present invention is a composition comprising D-galactose in association with coenzyme Q10 for orobuccal absorption for use as a food supplement or functional food or food for special medical purposes or medicament, in the treatment of chronic-degenerative diseases associated with an altered mitochondrial function.

According to the present invention with the expression formulations for "sublingual" administration it is intended tablets, capsules, bars, lozenges or granules having size so small (generally not exceeding 9-10 mm diameter) that can be placed in the sublingual cavity of the mouth, whereas with the expression formulations for "buccal", "peribuccal or orobuccal" administration it is intended tablets, capsules, bars, lozenges or granules having a greater size, which are dissolved or chewed in the oral cavity.

In both cases (sublingual or orobuccal administration) the delivery system consists in placing a given product (food supplement or functional food or food for special medical purposes or medicament) inside the oral cavity and waiting until it is dissolved after a short time, generally a few minutes; it is a form of a very fast intake, wherein the product comes into contact with the mucous membrane, and spreads, dissolving, even in the epithelium under the tongue, area full of blood vessels, entering into circulation, i.e. in the blood, much faster than the oral administration. Such an effect is explained in view of the fact that the oral haematic system discharges the active ingredient in the superior vena cava thus avoiding the portal venous system responsible of the known effect of first pass absorption splanchnic, thereby improving systemic bioavailability.

The composition comprising D-galactose in association with coenzyme Q10 according to the present invention is intended as complementary therapy, i.e. in association with drugs that the physician may prescribe according internationally recognized clinical protocols for the therapeutical treatment of the same pathologies.

According to the present invention, the expressions "treatment" or "treating" are intended to activities designed to cure, mitigate the symptoms or delay the progression of a disease or a pathological condition.

Furthermore, other active ingredients (o plant extract containing them) can be advantageously associated with D-galactose, according to the present invention and they are the following: *Curcuma longa L.* (titred in curcumin), L-theanin, *Plectranthus barbatus Andrews* (syn. *Coleus forskohlii* - titred in Forskohlina), asthaxantin, S-adenosyl-L-methionine, *Cucumis melo L.* (titred in superoxide dismutase), L-glutathione, NADH, homotaurine (or tramiprosate), membrane phospholipids (phosphoserine, phosphatidylserine, phosphatidylcholine), inositol, flavonoids, *Camelia sinensis L.* (titred in epigallocatechin gallate), choline, PABA (para-aminobenzoic acid), polyunsaturated fatty acids Omega-3 EPA and/or DHA, citicoline, melatonin, *Griffonia simplicifolia baill.*(titred in 5-HTTP - 5-hydroxy-tryptophan), GPC (Glycerylphosphorylcholine), GPE (Glycerylphosphorylethanolamine), lutein, zeaxanthin, *Vaccinium myrtillus L., Oxycoccus palustris pers., Brassica oleracea L.* (titred in sulforaphane), DHEA (dehydroepiandrosterone), *Camelia sinensis L.* (titred in GABA-gamma-aminobutyric acid), acetyl L-carnitine, N-acetyl-cysteine, polyunsaturated fatty acids Omega-6, palmitoylethanolamide (PEA), hypericum (*Hypericum perforatum*), *Bacopa monnieri, Escolzia, Magnolia, Passiflora,* arginine, citrulline, lipoic acid, hydroxytyrosol, oleuropein or *Ginkgo biloba* or mixtures thereof.

Therefore, a further object of the present invention are orosoluble and/or effervescent formulations for sublingual, or buccal or orobuccal or peribuccal use, containing D-gal and coenzyme Q10 in combination with physiologically acceptable excipients.

The orosoluble and/or effervescent compositions of the present invention are preferably characterized by the fact of having a high palatability, which guarantees an easy oral administration of the same. Said high palatability (meant as very pleasant taste) also determines a lower impulse to swallow, which helps to improve the absorption rate and bioavailability of the active ingredient due to a longer period of permanence of the composition in the oral cavity.

The orosoluble and/or effervescent compositions of the present invention can therefore be formulated in tablets, capsules and/or granules, preferably in the form of an orosoluble and/or effervescent tablet.

According to the present invention the term "orosoluble" refers to a composition capable of melting immediately, releasing the active ingredient contained therein when in contact with the oral mucosa.

In this way, the active ingredient can be absorbed directly into the oral mucosa, thus bypassing the hepatic system. According to the invention, the term orosoluble is therefore used to indicate compositions to be introduced in the oral cavity.

The compositions of the present invention can be formulated with further excipients. With the expression "excipient", it is meant here to refer to conventional type excipients, i.e. inert compounds with respect to the active ingredient and to the pharmaceutical form. Examples of different classes of these ingredients are: diluents (compounds added when the mass of the active ingredient is not sufficient for the preparation of the composition); lubricants (which prevent the powders to adhere to mechanical parts during the manufacturing process); aggregating agents (compounds which increase the cohesion of the powders); dyes (used to improve the presentation of some pharmaceutical forms, such as capsules, or to classify them according to the therapeutic category of affiliation, or to distinguish them from other similar products); sweeteners or flavourings (added to improve the organoleptic characteristics of the products); antioxidants-antimicrobials (used to prolong the shelf life of the product); gliding agents (used to improve the flow properties of the granules or powder and thus to facilitate the filling of the matrix in a homogeneous manner, allowing to achieve uniform tablets by weight); solubilizing agents (for favouring the solubilisation of the composition); pH regulating agents.

The excipients of the conventional type most commonly used are: lactose, glucose, saccharose, mannite (or mannitol), kaolin, talc, bentonite, titanium dioxide, xylitol, maltitol, sorbitol, sucralose, acesulfame K, aspartame, neohesperidin, fructose, dextrose, maltose, "spray-dried" malt, aspartate sodium, maltodextrin, sodium chloride, hydroxypropylmethylcellulose, erythritol, citrus extract, silica gel, vegetable fibres (as for example, the pea fibre), flavouring agents and aromas, such as mint flavour (peppermint, spearmint, fresh mint), anethole of star anise, vanilla, sage, liver, chicken, grapefruit, peach, orange, lemon or lime, sodium glutamate and fish flour.

To obtain the orosoluble for, the compositions of the invention are for example formulated using the classes of excipients described above.

More preferably, the orosoluble compositions according to the invention are formulated with magnesium oxide, magnesium hydroxide, alginic acid, stearic acid, hydrogenated vegetable oils (palm, oleic, behenic), cocoa butter, cocoa mass, xylitol, maltitol, sorbitol, mannitol, sucralose, acesulfame K, cyclamate, aspartame, sucrose, neohesperidin, fructose, dextrose, maltose, spray dried malt, aspartate sodium, maltodextrin, inositol, inulin, chitosan, beer yeast or a mixture of two or more of the foregoing excipients. In particular, in order to obtain the orosolubile form, said excipients are present in the compositions of the invention in an amount from 20% to 95% by weight, relative to the total weight of the formulation.

According to one embodiment of the invention, the D-galactose composition formulated for buccal, peribuccal or orobuccal administration, or, in the alternative, for sublingual administration, comprises at least the following excipients: mannitol, microcrystalline cellulose, polyvinylpyrrolidone, stearic acid, silicon dioxide, magnesium stearate, sucrose ester, hydroxypropylcellulose and polyethylene glycol.

According to a particular aspect of the present invention, the D-galactose composition having orobuccal absorption comprises one or more of the following excipients: mannitol, maltodextrin, microcrystalline cellulose, polyvinylpyrrolidone, stearic acid, silicon dioxide, magnesium stearate, sucrose ester, hydroxypropylcellulose, polyethylene glycol, sucralose and neohesperidin.

According to another particular aspect of the present invention, the D-galactose orobuccal absorption composition comprises one or more of the following excipients: mannitol, sodium bicarbonate, citric acid, microcrystalline cellulose, polyvinylpyrrolidone, stearic acid, silicon dioxide, magnesium stearate, sucrose ester, hydroxypropylcellulose, polyethylene glycol, saccharin and acesulfame K.

According to a further particular aspect of the present invention, the D-galactose orobuccal absorption composition comprises one or more of the following excipients: mannitol, xylitol, stearic acid, microcrystalline cellulose, polyvinylpyrrolidone, silicon dioxide, magnesium stearate, sucrose ester, hydroxypropylcellulose, polyethylene glycol, sucralose and hesperidin.

According to the invention, the term "effervescence" refers to compositions able to develop carbon dioxide when in contact with water and / or with the buccal environment, in the presence of saliva and are divided into:
- A: slightly effervescent buccal tablets: they are able to develop a slight effervescence able to ensure high palatability associated with a dissolution time of 10 minutes.
- B: traditional effervescent tablets: to be dissolved in water and to be swallowed in small sips.

To obtain the traditional effervescent form, the compositions according to the invention are formulated with citric acid, tartaric acid, adipic acid, monosodium phosphate, alginic acid or a mixture thereof, for the acid part, and magnesium hydroxycarbonate, sodium bicarbonate or potassium or a mixture thereof, for the development of carbon dioxide.

To obtain slightly effervescent formulations, a quota of citric acid will be added to obtain the effervescence in moderate amounts (slightly effervescent). In particular, said citric acid is added in slightly effervescent sublingual tablets in an amount ranging from 5% to 40%, preferably from 7% to 35% by weight, with respect to the total weight of the composition.

The compositions of the present invention can therefore be formulated in orosoluble form, effervescent form, or a combined orosolubile and effervescent form.

The term "high palatability", according to the present invention, refers to a composition having a particularly pleasant taste, which can be easily administered orally regardless of the fact that the active ingredients contained therein may have an unpleasant taste, bitter and/or sour.

The intake of D-galactose, for example associated with two antioxidant molecules such as ubiquinone (coenzyme Q10) and reduced glutathione, presents a target of neuro-protection based on the "metabolic" role of myelin. In practice, D-galactose is a nutrient exclusive of ectopic breathing systems, such as myelin, favoring, as occurring inside the mitochondrial structures, cellular respiration, that is the aerobic glycolytic metabolism.

With the expression "chronic degenerative diseases associated with an altered mitochondrial function" it is intended to include those pathologies, in which there is a block of cellular respiration to the advantage of a state of anaerobiosis, which represents the biochemical substrate favorable for the onset of oxidative stress with the progression of the "low-grade inflammation", anteroom of chronic degenerative processes. In other words, it is meant to refer to all degenerative diseases of the central (CNS) and peripheral (PNS) nervous system, where the appearance of demyelination or an impairment of nerve transmission functionality prevail in the clinical symptomatology. In particular, the expression "chronic degenerative diseases associated with an altered mitochondrial function" is meant to refer to all degenerative diseases of the central (CNS) and/or peripheral (PNS) nervous system associated with visual abnormalities and/or cerebral deficits with cognitive disabilities.

With the expression "chronic-degenerative diseases associated with altered mitochondrial function" it is also meant to encompass all clinical states, which comprehend a slowing down of the cerebral function, as in the proclaimed pre-senescence and senile states, and in ocular pathologies, where the neurological component (and in particular the involvement of rods and cones of retina) is the etiological cause of the alteration of the vision.

More specifically, it is intended, for example, to refer to the following pathologies: Muscle-Eye-Brain disease (MEB disease), glaucoma, multiple sclerosis (MS), amyotrophic lateral sclerosis (ALS), demyelinating diseases (autoimmune based), degenerative neuropathies based on metabolism (diabetes, vitamin deficiencies), traumatic neuropathies or entrapment syndromes, psycho-motor delays in childhood, cognitive disorders of senescence in the initial phase, elderly-related macular degeneration, diabetic retinopathy, senile dementia, Alzheimer's disease, subacute benign polyradiculoneuropathy or Guillian Barre syndrome, acute disseminated encephalomyelitis (post-infectious encephalitis), adrenoleukodystrophy and adrenomyeloneuropathy, Leber's hereditary optic atrophy and mitochondrial diseases, myelopathy, HTLV-associated (associated with Human T-cell Lymphotrophic Virus), type I and II sensory and motor neuropathy (disease Charcot-Marie-Tooth disease, peroneal muscular atrophy) or type III sensory and motor neuropathy, (neuropathy interstitial hypertrophic, Dejerine-Sottas disease).

It is object of the present invention a functional food containing the composition of the present invention in one of the pharmaceutical forms previously described.

With the expression "functional food" it is intended to include the foods characterized by additional effects due to the presence of components (generally non nutrient) naturally present or added, which interact more or less selectively with one or more physiological functions of the organism (biomodulation), bringing positive effects on health maintenance and/or disease prevention. A food can be considered "functional", if its beneficial influence on one or more functions of the body is sufficiently demonstrated, in addition to adequate nutritional effects, so as to be relevant to a state of well-being and health or reduce the risk of a disease. The beneficial effects could include both maintaining as well as promoting a state of well-being or health and/or a reduced risk of a pathological process or disease (see Diplock A.T. et al: Scientificconcepts of functionalfoods in Europe: ConsensusDocument, British Journal of Nutrition 1999, 81 (Suppl. 1),S1-S27).

It a further object of the present invention a food supplement containing the composition of the present invention in one of the pharmaceutical forms previously described.

With the expression "food supplement (FS)", it is meant a food product intended to supplement the common diet and that constitutes a concentrated source of nutrients, such as vitamins and minerals, or of other substances having a nutritional or physiological effect, in pre-dosed forms (also see Directive 2002/46/CE dated 10 June 2002 and Italian DLG dated 21 May 2004 n. 169 art. 2).

Furthermore, it is object of the present invention a food for special medical purposes (FSMP) containing the composition of the present invention in one of the pharmaceutical forms previously described.

With the expression "food for special medical purposes (FSMP)", it is intended as defined in the EU Regulation (N° 609/2013), as well as in the Guidelines issued by the EFSA (European Food Safety Authority) entitled "Scientific and technical guidance on food for special medical purposes in the context of Article 3 of Regulation (EU) No 609/2013" - EFSA Journal 2015;13(11):430. In particular, this regulatory definition is divided into three points:
1) a food expressly processed or formulated and intended for the dietary management of patients, including infants, to be used under medical supervision;
2) intended for exclusive or partial feeding of patients with limited, disturbed or altered capacity to take, digest, absorb, metabolize or eliminate common foodstuffs or certain nutrients contained therein or metabolites, or with other specific nutritional needs determined by clinical conditions;
3) whose dietary management cannot be carried out exclusively by modifying the common diet.

In order to be proposed and appointed as FSMP, a product must meet the three points of the previous definition.

For the purposes of the distinction with FS it should also be pointed out that, while the latter products are in conformity with regulatory definition of EU Directive 2002/46, even if they only have beneficial "physiological effect" for the intake of substances other than nutrients without sharing in the constitution of the fodd portion (e.g. products based on plant extracts), the FSMP must necessarily have a "nutritional" role, as constituents of a food portion designed to meet the nutritional requirements of patients with specific nutritional vulnerability.

The production process of the composition of the present invention is carried out using the well known technologies and operative conditions. In general, the process comprises mixing the active ingredients (or the plant extracts contained therein) together with excipients and other necessary components in a mixer, in order to obtain the desired composition.

For example, the process schematically comprise the following steps: weighting the active ingredients and the necessary excipients in order to obtain the desired release, mixing all these components in a mixer (for example with a rotating cochlea), compressing the mixture obtained and subsequent painting with transparent or colored dye. In case of poorly flowing powders a pre-compression may be carried out, before the compression, and subsequent dry granulation or alternatively a wet granulation can be implemented.

The process for preparing the orosoluble or sublingual tablets, also effervescent, schematically comprises the same steps previously listed, with the arrangement that the production steps must be conducted in an environment with controlled temperature and humidity (generally a temperature around 25° C and a humidity below 30%) and that agents, which induce a fast or immediate release of at least one of the active ingredients will have to used. For example, processes for the preparation of the formulations of the present invention are schematically described in the international patent application WO2010/089674 (see pages 11-12, Schemes A, B and C).

In particular, the preparation of tablets, granules, capsules, bars or orobuccal lozenges, Scheme A comprises the following steps:
1. Weighing the active ingredients and the excipients;
2. Mixing (rotating cochlea mixer);
3. Final compressing or directly packaging the mixture obtained in sachets or in capsules.

For the preparation of both mono- and multi-layer tablets, granules, orobuccal capsules, tablets and lozenges, Scheme B comprises the following steps:
1. Weighing the active ingredients and the excipients;
2. Mixing (rotating cochlea mixer);
3. Compressing;
4. Granulating the resulting tablets through an oscillating granulator;
5. Finally compressing by using appropriate multilayer tabletting machines or directly packaging the granulate obtained in step 4 in sachets or in capsules.

For the preparation of both mono- and multi-layer tablets, granules, orobuccal capsules, tablets and lozenges, Scheme C comprises the following steps:
1. Weighing the active ingredients and the excipients;
2. Mixing the active ingredients and the excipients in moist/warm conditions;
3. Granulating, after cooling the dough, and adding other excipients;
4. Finally compressing by using appropriate multilayer tabletting machines or u directly packaging the mixture obtained in step 3 in sachets or in capsules.

An example of an orosoluble tablet formulation according to the present invention is shown in the following Table 1.

**Table 1**

| **Ingredient / excipient** | **mg/tablet** |
|---|---|
| Galactose | 500 |
| Mannitol | 300 |
| Coenzyme Q10 | 50 |
| Pure Reduced Glutathione | 50 |
| Maltodextrin | 27.3 |
| Microcrystalline cellulose | 26 |
| Kollidon® | 22.4 |
| Stearic acid | 20 |
| Silicon dioxide | 12 |
| Flavourings | 10 |
| Magnesium stearate | 10 |
| Sucrose esters | 10 |
| Klucel™ | 5.1375 |
| Carbowax™ | 5 |
| Sucralose | 2 |
| Neohesperidine | 0,4 |
| (Total weight) | 1,050.2375 |

An example of a formulation in a slightly effervescent tablet not comprising coenzyme Q10 (reference formulation) is shown in the following Table 2

**Table 2**

| **Ingredient / excipient** | **mg/tablet** |
|---|---|
| Galactose | 500 |
| Mannitol | 300 |
| Sodium bicarbonate | 250 |
| Citric acid | 70 |
| Microcrystalline cellulose | 26 |
| Kollidon® | 25 |
| Stearic acid | 20 |
| Silicon dioxide | 12 |
| Flavourings | 10 |
| Magnesium stearate | 10 |
| Sucrose ester | 10 |
| Klucel™ | 5 |
| Carbowax™ | 5 |
| Saccharin | 5 |
| Acesulfame K | 2.5 |
| (Total weight) | 1,250.5 |

An example of formulation in orosoluble tablets "fast-slow" not comprising coenzyme Q10 (reference formulation) is reported in the following Table 3.

**Table 3**

| **Ingredient / excipient** | **mg/tablet** |
|---|---|
| Galactose | 500 |
| Mannitol | 500 |
| Xylitol | 500 |
| Stearic acid | 80 |
| Homotaurine | 50 |
| Microcrystalline cellulose | 92 |
| Kollidon® | 20 |
| Silicon dioxide | 12 |
| Flavourings | 10 |
| Magnesium stearate | 10 |
| Sucrose ester | 10 |
| Klucel™ | 5 |
| Carbowax™ | 5 |
| Sucralose | 5.5 |
| Neohesperidine | 0.5 |
| (Total weight) | 1,800 |

Some examples of the present invention are provided below, that will refer to one or more Figures.

### DESCRIPTION OF THE FIGURES

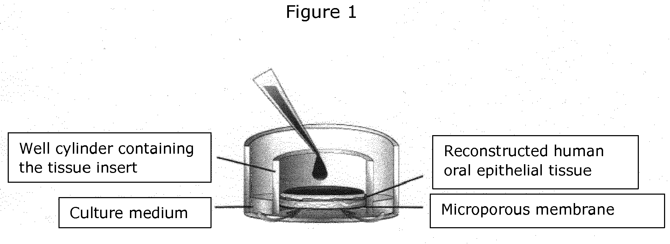

Figure 1. Figure 1 schematically represents the experimental model of reconstructed human oral epithelial tissue used in Example 2. In particular, the external cavity is one of the wells of a well plate. The inner cavity is a cylinder open at its ends, which contains in its interior an insert with the reconstructed human oral epithelial tissue on a microporous membrane formed by a polycarbonate mesh filter. The culture medium is located at the base of the well and is in contact only with the microporous membrane and therefore only with the lower part of the reconstructed tissue. The sample to be tested was instead applied from above on the reconstructed tissue.

### EXAMPLES

### Example 1

### Preparation of an orosoluble tablet containing D-galactose

A tablet was prepared following the Scheme A previously reported. Therefore, the ingredients and the excipients reported in the Table were weighed in the amounts reported in Table 1. Such ingredients and excipients were mixed using a rotating cochlea mixer. The mixture thus obtained was loaded in a tabletting machine, in order to obtain the tablet.

### Example2

### In vitro evaluation of the penetrating capacity of D-galactose contained in an orosoluble tablet through the reconstructed human oral tissue

For the experiment, the orosoluble tablet obtained in Example 1 was used. The scope of the experiment was to evaluate the penetrating capacity of D-galactose, present in the orosoluble tablet, through reconstructed human oral epithelium (HOES5, commercialized by SkinEthicLaboratories, France)

0.5 cm² of reconstructed human oral epithelium were used for each experiment. Said tissue was supplied by SkinEthicLaboratories on a porous membrane constituted by a polycarbonate filter mesh inserted inside a cylinder to be introduced into a well. To carry out the experiments said cylinders have been arranged in a well plate with a diameter suitable to allocate the cylinders and said tissue contained in each well was kept in maintenance medium (SMM SkinEthicMaintenance Medium). The maintenance medium was in contact only with the lower part of this reconstructed tissue. A schematic representation of this experiment is shown in Figure 1.

The tablet to be tested was preliminarily dissolved in artificial saliva and applied on the surface of the reconstructed oral human tissue, in order to evaluate the concentrations of D-galactose, absorbed and released by the tissue in the culture medium to the following experimental times:
- T10: 10 minutes after applying the product
- T20: 20 minutes after applying the product
- T30: 30 minutes after applying the product

At the end of the experimental period, the vitality of the treated oral tissue cells was evaluated, in order to identify a possible effect of contact soreness of the oral mucosa. Finally, it was also evaluated the amount of D-galactose still present in the structure of the tissue and not released into the culture medium.

The artificial saliva had the composition reported in the following Table 4 (Alshali et al., (2015) J. Dent. 43:1511-1518, Table 1).

**Table 4**

| **Compound** | **Concentration (g/l) in distilled water** | **Supplier** |
|---|---|---|
| Methyl 4-hydroxybenzoate | 2.0 | Sigma-Aldrich |
| Sodiumcarboxymethylcellulose (CMC) | 10.0 | Sigma-Aldrich |
| Potassium chloride (KCl) | 0.625 | Fluka |
| Calcium chloride (CaCl₂) | 0.166 | Sigma-Aldrich |
| Potassium dihydrogen phosphate (KH₂PO₄) | 0.326 | Merck |
| Magnesium chloride (MgCl₂) | 0.059 | Sigma-Aldrich |
| Potassium hydroxide (KOH) | q.s. until pH 6.75 | Fluka |

The solution to be tested was prepared considering that the absorbent buccal surface corresponds to approximately 200 cm² and has a rich vascular network and that during the day non-homogeneous saliva can be produce, of about 1,500 ml, with an average of 10 ml/hour up to 250 ml/hour under stimulation or 0 ml/hour during the sleep.

According to these data, the average volume of saliva produced was used to obtain the test D-galactose solution, as follows:
- 1 g of powder (containing 500 mg of Galactose) was dissolved in 10 ml of artificial saliva at 37°C for 1 hour, obtaining a concentration of D-galactose equal to 50 mg/ml.

### Penetration test

A 25 µl volume solution (equal to 10 ml of saliva on a 200 cm² surface), containing 1.25 mg/ml of D-galactose, was applied on the surface of the tissue, in replicate, for 10 minutes (T10), 20 minutes (T20) and 30 minutes (T30). At the end of each experiment, the culture medium was collected for the next dosage of D-galactose.

After the last sample, one of the tissues remained was used for the evaluation of the tissue vitality.

### D-galactose dosage

The dosage of the D-galactose was performed by carrying out a test based on a redox reaction with 3,5-dinitrosalicylic acid (DNS) [Stasiak-Różańska L. et al., (2011), Acta Sci. Pol. Technol. Aliment. 10(1):35-49.]: the oxidation of the aldehyde group or ketone group present in the reducing sugars was exploited; DNS was simultaneously reduced, in alkaline conditions, to 3-amino-5-nitrosalicilico, a reddish compound that absorbs at a wavelength (λ) 540 nm.

### Evaluation of the tissue vitality

The vitality of the treated tissue was evaluated by MTT test [Mosmann T (1983), J Immunol. Methods 65(1-2):55-63.], based on the reduction of the key reagent 3-[4,5-dimetilthiazol-2-yl]-2,5-diphenyl tetrazolium bromide, applying the modified protocol as indicated below:

### Incubation in the MTT solution

The wells of a 24-well plate was filled with 300 µl of a MTT solution

The treated tissues were transferred into the wells and incubated for 3 hours (+/- 5 minutes) at 37°C, 5% CO₂ and 95% humidity.

### Formazan extraction

A new 24-well plate was filled with 800 µl of isopropanol for each well.

At the end of 3 hours incubation (± 5 minutes) in the MTT solution, the treated tissues were transferred in the isopropanol solution. 700 µl of isopropanol were added on the treated tissue and was incubated for 2 hours (± 5 minutes) at room temperature under stirring (about 150 rpm) for the formazan extraction .

### Measurement of the optical density (OD)

At the end of 2 hours incubation (± 5 minutes), the tissues and the polycarbonate filters were perforated, so as to mix and homogenize the extraction solution.

From each well thus treated 200 µl of extraction solution were collected (= 3 wells for each tissue or 3 replicates per tissue), which were transferred in a 96-well plate. The optical density of the wells was then read at (λ) 540 nm.

### Data analysis

According to the classification indicated in the validation documents of the methods employed by the tissue supplier, the vitality of the treated tissues with the test sample and the positive control was calculated with respect to the negative control and compared with the following criteria:
Tissue average vitality ≤ 50%, IRRITATING (I)
Tissue average vitality > 50%, NOT IRRITATING (NI)

### Results

In Table 5 are reported the D-galactose dosages contained in the orosoluble tablet, present in the oral tissues. The results are expressed as D-galactose amount in mg (mean ± standard deviation) found in the culture medium. Also the partial (with respect to the experimental time monitored) and total absorption percentages (%) were calculated and reported in Table 5.

**Tabella 5**

| **Sample** | **Concentration D-gal (mg/ml)** | **SD** | **% Partial absorption** | **% Total absorption** |
|---|---|---|---|---|
| Medium at T10 | 0.541 | 0.009 | 43 | 43 |
| Medium at T20 | 0.081 | 0.04 | 6.49 | 49.75 |
| Medium at T30 | 0.013 | 0.02 | 1.07 | 50.82 |
| Control | 0 | 0 | - | - |

| | | | | |
|---|---|---|---|---|
| Legend: SD = Standard deviation of the mean; Control = untreated buffer and tissue | | | | |

The analysis of the results obtained shows a penetration of D-galactose, contained in the tested product, through the oral mucosa reconstructed *in vitro,* which reaches the 43% within the first 10 minutes of application and reaches to about 51% within 30 minutes.

If a traditional tablet (swallowable, non-buccal) were subjected to the same experiment, you would have obtained the same results that were obtained with the control, as the traditional tablet would not dissolve in artificial saliva.

In Table 6 the results of the MTT test performed on human oral tissue HOES5 for the evaluation of the tolerability of the product for the oral mucosa are reported.

**Table 6**

| **Sample** | **OD 540nm** | **Tissue vitality** | **Classification** |
|---|---|---|---|
| Treated tissue | 0.807 | 99% | Not irritating |
| Not-treated tissue | 0.795 | 100% | Not irritating |

The performed MTT test does not show any sign of irritation to the oral mucosa resulting from the application of the tested composition dissolved in artificial saliva

### Conclusions

In accordance with the test and the experimental conditions used, it was concluded that: D-galactose (500 mg), contained in the orobuccal formulation as a tablet showed penetration capacity through the oral reconstructed epithelial tissue.

### Example 3

### Clinical Case Report 1

A male patient, 28 years old, with severe "leukodystrophy" from birth. The diagnosis in 2013 was definitely corrected in "MEB disease - Muscle-Eye-Brain disease". It is a genetic disorder with muscular dystrophic degeneration and visual abnormalities (myopia) and severe cerebral deficits with major cognitive disabilities.

The patient came to our observation in October 2012. He had an important presence of seizures (even 5 per day) despite assumption of anticonvulsants.

The neurological picture was characterized by aphasia, obsessive-compulsive attitude (fixation on figures of children and women from whom he is attracted). Sometimes he manifested aggressively, but never disturbing.

He was subjected to immunomodulatory therapy with the use of low dose cytokines, thus obtaining a marked improvement in the quantity and quality / duration of seizures (reduced to 2 per week, of low intensity and duration of max 30 seconds).

However, this therapy did not have any impact on the intellectual framework and on his attitudes.

From December 2015, the patient started a treatment with the product described in Example 1 at the dosage of two tablets per day.

Even after one month of treatment, parents observed a change in the patient's state of mind: a decrease of obsessive-compulsive attitudes, a greater understanding and participation to reality (e.g., he laughed, when the context was comic, he grieved, when the context was dramatic or sad) and the patient was even able to articulate short sentences. This change, according to parents and educators who follow him, was a remarkable and unexpected change, also because it kept constant over time.

We are thinking at the possibility of increasing the daily dosage of the composition of Example 1, in order to further improve the clinical picture.

### Example 4

### Clinical Case Report 2

A 65-year-old female patient, who was diagnosed with open-angle acute primary glaucoma with sudden vision loss in the right eye (OD), was presented. Intraocular pressure (IOP), evaluated with applanation tonometry, was at the onset of 28 mmHg (right eye) and 22 mmHg (left eye). There was no vitreous or choroidal pathology, but discopathy at OD. The visual field had widespread defects in both eyes, more accentuated in the OD. A medical treatment for ophthalmic IOP was initiated with ophthalmic ophthalmic beta-toxolol, but after 4 months of treatment, field of vision and acuity was not improved.

A neuroprotective treatment (in addition to topical therapy with betaxolol) was then performed, with an integration of two tablets prepared as in Example 1 per day (after the evening meal) for 3 months. Following this treatment, automated perimetry showed significant improvement, even subjectively acknowledgeable. These results were maintained after 4 months of discontinuation of treatment. Patient was advised to complete another cycle of 2 months and a 2-month suspension and repeat these cycles at least for one year.

## Claims

1. A composition formulated for buccal, peribuccal or orobuccal administration, or, in the alternative, for sublingual administration, comprising D-galactose in association with coenzyme Q10 and, optionally, reduced glutathione, as active ingredients, together with pharmaceutically acceptable excipients, for use as food supplement or functional food or food for special medical purposes or as medicament.

2. The composition for use according to claim 1, which comprises at least one additional active ingredient or plant extract selected from the group comprising: *Curcuma longa L.* (titrated in curcumin), L-theanine, *Plectranthus barbatus Andrews* (syn. *Coleus forskohlii-* titrated in forskolin), astaxanthin, S-adenosyl-L-methionine, *Cucumismelo L.* (titrated in superoxide dismutase), L-glutathione, NADH, homotaurine (tramiprosate), membrane phospholipids (phosphoserine, phosphatidylserine, phosphatidylcholine), inositol, flavonoids, *Camelia sinensis L.* (titrated in epigallocatechin gallate), choline, PABA (para-amino benzoic acid), polyunsaturated fatty acids Omega-3 EPA/DHA, citicoline, melatonin, *Griffoniasimplicifoliabaill.* (titrated in 5-HTP - 5-hydroxytryptophan), GPC (glycerylphosphorylcholine), GPE (Glyceryl Phosphoryl ethanolamine), lutein, zeaxanthin, *Vacciniummyrtillus L., Oxycoccuspalustris pers., Brassica oleracea L.* (titrated in sulforaphane), DHEA (dehydroepiandrosterone), *Camelia sinensis L.* (titrated in GABA - gamma-aminobutyric acid), acetyl-L-carnitine, N-acetylcysteine, polyunsaturated fatty acids Omega-6 and palmitoylethanolamide (PEA) Hypericum (*Hypericumperforatum*), *Bacopamonnieri, Escolzia, Magnolia, Passiflora,* arginine, citrulline, lipoic acid, hydroxytyrosol, oleuropein, *Ginkgo biloba,* together with pharmaceutically acceptable excipients.

3. The composition for use according to any of the preceding claims, for the treatment of chronic degenerative diseases associated with altered mitochondrial function.

4. The composition for use according to any of the preceding claims, for the treatment of all degenerative diseases of the central (CNS) and/or peripheral (PNS) nervous system associated with visual abnormalities and/or cerebral deficits with cognitive disabilities.

5. The composition for use according to any of the preceding claims, where the chronic-degenerative disease is selected from the group comprising: Muscle-Eye-Brain disease (MEB disease), glaucoma, multiple sclerosis (MS), amyotrophic lateral sclerosis (ALS), demyelinating diseases (autoimmune), degenerative neuropathies on metabolic basis (diabetes, vitamin deficiencies), or traumatic neuropathies entrapment syndromes, psycho-motor delays in childhood, senescence cognitive disorders in the initial phase, the elderly macular degeneration, diabetic retinopathy, senile dementia, Alzheimer's disease, benign subacute polyradiculoneuropathy or Guillain-Barre syndrome, acute disseminated encephalomyelitis (postinfectious encephalitis) adrenoleukodystrophy and adrenomyeloneuropathy, Leber's hereditary optic atrophy and mitochondrial diseases, HTLV-associated myelopathy (associated with Human T-cell Lymphotrophic Virus), type I and II motor and sensory neuropathy (Charcot-Marie-Tooth disease, peroneal muscular atrophy) and type III motor and sensory neuropathy (interstitial hypertrophic neuropathy, Dejerine-Sottas disease).

6. The composition for use according to any of claims from 3 to 5, in which the treatment is intended as a complementary therapy, that is, in association with other drugs for the therapeutic treatment of the same pathology.

7. The composition for use according to any one of the preceding claims, in orosoluble and/or effervescent formulation.

8. The composition for use according to any of the preceding claims, comprising 0.5 g of D-galactose in association with 50 mg of coenzyme Q10 and, optionally, 50 mg of reduced glutathione.

9. The composition for use according to any of the preceding claims, comprising at least the following excipients: mannitol, microcrystalline cellulose, polyvinylpyrrolidone, stearic acid, silicon dioxide, magnesium stearate, sucrose ester, hydroxypropylcellulose and polyethylene glycol.

10. The composition for use according to any of the preceding claims, in form of tablet capsules, bars, dragees, pills, or granules.

11. A food supplement, which comprises the composition according to one of the preceding claims.

## Patentansprüche

1. Eine Zusammensetzung, formuliert für bukkale, peribukkale oder orobukkale Verabreichung, oder, alternativ, für sublinguale Verabreichung, umfassend D-Galactose zusammen mit dem Coenzym Q10 und, optional, reduziertem Glutathion als Wirkstoffe, zusammen mit pharmazeutisch annehmbaren Hilfsstoffen, zur Verwendung als Nahrungsergänzung oder als Functional Food oder als Lebensmittel für besondere medizinische Zwecke oder als Medikament.

2. Die Zusammensetzung "zur Verwendung", gemäß Anspruch 1, umfassend mindestens einen zusätzlichen Wirkstoff oder ein Pflanzenextrakt, ausgewählt aus der Gruppe bestehend aus: *Curcuma longa L.* (titriert in Curcumin), L-Theanin, *Plectranthus barbatus Andrews* (syn. *Coleus forskohlii* - titriert in Forskolin), Astaxanthin, S-Adenosyl-L-Methionin, *Cucumismelo L.* (titriert in Superoxiddismutase), L-Glutathion, NADH, Homotaurin (Tramiprosat), Membranphospholipiden (Phosphoserin, Phosphatidylserin, Phosphatidylcholin), Inositol, Flavonoiden, *Camelia sinensis L.* (titriert in Epigallocatechingallat), Cholin, PABA (p-Aminobenzoesäure), mehrfach ungesättigten Fettsäuren Omega-3 EPA/DHA, Citicolin, Melatonin, *Griffoniasimplicifoliabaill.* (titriert in 5-HTP - 5-Hydroxytryptophan), GPC (Glycerylphosphorylcholin), GPE (Glyceryl-Phosphoryl-Ethanolamin), Lutein, Zeaxanthin, *Vacciniummyrtillus L., Oxycoccuspalustris pers., Brassica oleracea L.* (titriert in Sulforaphan), DHEA (Dehydroepiandrosteron), *Camelia sinensis L.* (titriert in GABA - *γ*-Aminobuttersäure), Acetyl-L-Carnitin, N-Acetylcystein, mehrfach ungesättigten Fettsäuren Omega-6 und Palmitoylethanolamid (PEA) Hypericum (*Hypericumperforatum*), *Bacopamonnieri*, *Escolzia*, *Magnolia, Passiflora,* Arginin, Citrullin, Liponsäure, Hydroxytyrosol, Oleuropein, *Ginkgo biloba,* zusammen mit pharmazeutisch annehmbaren Hilfsstoffen.

3. Die Zusammensetzung "zur Verwendung", gemäß eines jeglichen der vorhergehenden Ansprüche, zur Behandlung von chronisch-degenerativen Erkrankungen assoziiert mit veränderter mitochondrialer Funktion.

4. Die Zusammensetzung "zur Verwendung", gemäß einem jeglichen der vorhergehenden Ansprüche, zur Behandlung von allen degenerativen Erkrankungen des zentralen (CNS) und/oder peripheren (PNS) Nervensystems, assoziiert mit visuellen Anomalien und/oder Hirnfunktionsstörungen mit kognitiven Einschränkungen.

5. Die Zusammensetzung "zur Verwendung", gemäß einem jeglichen der vorhergehenden Ansprüche, wobei die chronisch-degenerativen Erkrankung ausgewählt ist aus der Gruppe bestehend aus: Muskel-Auge-Gehirn-Krankheit (MEB Disease), Glaukom, multipler Sklerose (MS), amyotropher Lateralsklerose (ALS), demyelinisierenden Erkrankungen (autoimmun), degenerativen Neuropathien auf metabolischer Basis (Diabetes, Vitaminmangel) oder traumatischen Nervenkompressionssyndromen, psychomotorischen Verspätungen in der Kindheit, kognitiven Störungen der Seneszenz in der Anfangsphase, altersbedingter Makula-Degeneration, diabetischer Retinopathie, seniler Demenz, Alzheimer-Krankheit, benigner subakuter Polyradiculoneuropathie oder Guillain-Barre-Syndrom, akuter disseminierter Enzephalomyelitis (postinfektiöse Enzephalitis) Adrenoleukodystrophie und Adrenomyeloneuropathie, Leberscher Optikusatrophie und mitochondrialer Erkrankungen, HTLV-assoziierter Myelopathie (assoziiert mit humanem T-Zell-lymphotropem Virus), Typ I und II motorisch-sensibler Neuropathie (Morbus Charcot-Marie-Tooth, peroneale Muskelatrophie) und Typ III motorisch-sensibler Neuropathie (interstitielle hypertrophe Neuropathie, Dejerine-Sottas-Krankheit).

6. Die Zusammensetzung "zur Verwendung", gemäß einem jeglichen der Ansprüche von 3 bis 5, wobei die Behandlung als Komplementärtherapie zusammen mit anderen Medikamenten zur therapeutischen Behandlung für die gleiche Pathologie geplant ist.

7. Die Zusammensetzung "zur Verwendung", gemäß einem jeglichen der vorhergehenden Ansprüche, in orolöslicher und/oder Brauseformulierung.

8. Die Zusammensetzung "zur Verwendung", gemäß einem jeglichen der vorhergehenden Ansprüche, umfassend 0.5 g D-Galactose zusammen mit 50 mg des Coenzyms Q10 und, optional, 50 mg reduziertem Glutathion.

9. Die Zusammensetzung "zur Verwendung", gemäß einem jeglichen der vorhergehenden Ansprüche, umfassend mindestens die folgenden Hilfsstoffe: Mannitol, mikrokristalline Cellulose, Polyvinylpyrrolidon, Stearinsäure, Siliciumdioxid, Magnesiumstearat, Saccharosester, Hydroxypropylcellulose und Polyethylenglycol.

10. Die Zusammensetzung "zur Verwendung", gemäß einem jeglichen der vorhergehenden Ansprüche, in Form von Tabletten-Kapseln, Stangen, Dragees, Tabletten oder Granulaten.

11. Eine Nahrungsergänzung, die die Zusammensetzung gemäß einem der vorhergehenden Ansprüche umfasst.

## Revendications

1. Composition formulée pour l'administration buccale, péribuccale ou orobuccale ou, en alternative, pour l'administration sublinguale, comprenant du D-galactose en association avec la coenzyme Q10 et, éventuellement, du glutathion réduit, en tant qu'ingrédients actifs, conjointement avec des excipients pharmaceutiquement acceptables, à utiliser complément alimentaire, aliment fonctionnel ou aliment destiné à des fins médicales spéciales ou à titre de médicament.

2. Composition à utiliser selon la revendication 1, qui comprend au moins un ingrédient actif supplémentaire ou un extrait de plante choisi dans le groupe comprenant : *Curcuma longa L.* (titré en curcumine), L-théanine, *Plectranthus barbatus Andrews* (syn. *Coleus forskohlii* - titré en forskoline), astaxanthine, S-adénosyl-L-méthionine, *Cucumismelo L.* (titré en superoxyde dismutase), L- glutathion, NADH, homotaurine (tramiprosate), phospholipides membranaires (phosphosérine, phosphatidylsérine, phosphatidylcholine), inositol, flavonoïdes, *Camelia sinensis L.* (titré en gallate d'épigallocatéchine), choline, PABA (acide para-amino benzoïque), acides gras polyinsaturés oméga-3 EPA/DHA, citicoline, mélatonine, *Griffoniasimplicifoliabaill.* (titré en 5-HTP - 5-hydroxytryptophane), GPC (glycérylphosphorylcholine), GPE (glycérol phosphoryl éthanolamine), lutéine, zéaxanthine, *Vacciniummyrtillus L., Oxycoccuspalustris pers., Brassica oleracea L.* (titré en sulforaphane), DHEA (déhydroépiandrostérone), *Camelia sinensis L.* (titré en GABA - acide gamma-aminobutyrique), acétyl-L-carnitine, N-acétylcystéine, acides gras polyinsaturés oméga-6 et palmitoyléthanolamide (PEA) Hypericum (*Hypericumperforatum*), *Bacopamonnieri, Escolzia, Magnolia, Passiflora,* arginine, citrulline, acide lipoïque, hydroxytyrosol, oleuropéine, *Ginkgo biloba,* ainsi que des excipients pharmaceutiquement acceptables.

3. Composition à utiliser selon l'une des revendications précédentes, pour le traitement de maladies dégénératives chroniques associées à une altération de la fonction mitochondriale.

4. Composition à utiliser selon l'une des revendications précédentes, pour le traitement de toutes les maladies dégénératives du système nerveux central (SNC) et/ou périphérique (SNP) associées à des anomalies visuelles et/ou des déficits cérébraux présentant des déficiences cognitives.

5. Composition à utiliser selon l'une des revendications précédentes, dans lequel la maladie dégénérative chronique est choisie dans le groupe comprenant : la maladie muscle-oeil-cerveau (maladie de Santavuori), le glaucome, la sclérose en plaques (MS), la sclérose latérale amyotrophique (SLA), maladies démyélinisantes (auto-immunes), neuropathies dégénératives métaboliques (diabète, carences en vitamines) ou neuropathies traumatiques syndromes canalaires, retards psychomoteurs pendant l'enfance, troubles de la sénescence cognitive en phase initiale, dégénérescence maculaire chez les personnes âgées, rétinopathie diabétique, démence sénile, maladie d'Alzheimer, polyradiculonévrite subaiguë bénigne ou syndrome de Guillain-Barré, encéphalomyélite aiguë disséminée (encéphalite postinfectieuse) adrénoleucodystrophie et adrénomyélonéopathie, Atrophie optique héréditaire de Leber et maladies mitochondriales, myélopathie associée au HTLV (associée au virus humain T lymphotrophe), neuropathie motrice et sensorielle de type I et II (maladie de Charcot-Marie-Tooth, atrophie musculaire péronière) et neuropathie motrice et sensorielle de type III (neuropathie hypertrophique interstitielle, maladie de Dejerine-Sottas).

6. Composition à utiliser selon l'une des revendications 3 à 5, dans lesquelles le traitement est destiné à une thérapie complémentaire, c'est-à-dire en association avec d'autres médicaments pour le traitement thérapeutique de la même pathologie.

7. Composition à utilizer Selon l'une des revendications précédentes, en formulation orosoluble et/ou effervescente.

8. Composition à utiliser selon l'une des revendications précédentes, comprenant 0,5 g de D-galactose en association avec 50 mg de coenzyme Q10 et, éventuellement, 50 mg de glutathion réduit.

9. Composition à utiliser selon l'une des revendications précédentes, comprenant au moins les excipients suivants : mannitol, cellulose microcristalline, polyvinylpyrrolidone, acide stéarique, dioxyde de silicium, stéarate de magnésium, ester de saccharose, hydroxypropylcellulose et polyéthylène glycol.

10. Composition à utiliser selon l'une des revendications précédentes, sous forme de comprimés, de capsules, de barres, de dragées, de pilules ou de granules.

11. Complément alimentaire comprenant la composition selon l'une des revendications précédentes.
